# EUROPEAN PATENT APPLICATION

(11) **EP 0 666 077 A1**
(43) Date of publication of application: **09.08.1995**
(21) Application number: 94922378.8
(22) Date of filing: 16.08.1994
(51) Int. Cl.: A61K 31/415, A61L 2/16, A01N 43/50

(54) **ANTI-MRSA COMPOSITION**

(30) Priority: 16.08.1993 JP 202323/93
(71) Applicant: MEIJI SEIKA KABUSHIKI KAISHA, Tokyo 104 (JP); BIOMATERIAL CO., LTD., Fukui 910 (JP)
(72) Inventor: YONETA, Toshio Meiji Seika Kabushiki Kaisha, Odawara-shi Kanagawa 250-01 (JP); FUJII, Naoyuki Biomaterial Co., Ltd., Fukui 910 (JP); OOISHI, Maki Meiji Seika Kabushiki Kaisha, Odawara.shi Kanagawa 250-01 (JP); IINUMA, Katsuharu Meiji Seika Kabushiki Kaisha, Odawara-shi Kanagawa 250-01 (JP); SATO, Atsuyuki Meiji Seika Kabushiki Kaisha, Odawara-shi Kanagawa 250-01 (JP); YASUDA, Kimiaki Biomaterial Co., Ltd., Fukui 910 (JP)
(74) Representative: Kyle, Diana
(86) International application number: JP9401353
(87) International publication number: WO9505171

(57) **Abstract**

An anti-methicillin-resistant *Staphylococcus aureus* (MRSA) composition containing an imidazole derivative represented by general formula (I), wherein R¹, R², R³ and R⁴ represent each independently hydrogen or halogen. Since the above compound has an anti-MRSA activity, it can provide a composition efficacious in preventing infection with MRSA and a textile product bearing the composition.

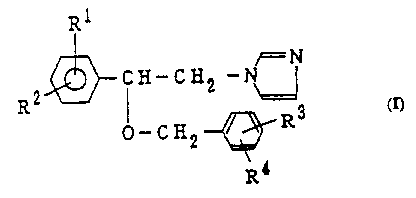

## Description

### BACKGROUND OF THE INVENTION

### Technical Field

The present invention relates to a composition for preventing the methicillin resistant Staphylococcus aureus (MRSA) infection, and also relates to fibrous products, paper products and sanitary goods which carry the composition.

### Prior Art

MRSA is resistant to most of antibiotics. This means no proper therapeutics against the infection from MRSA. Consequently, a patient once suffered from MRSA is difficult to be cured and easily fallen into a grave condition, so that medical investigators have paid attention to MRSA. The MRSA infection is mainly a nosocomial infection of compromised hosts having a decreased resistance during or after treatment. While the MRSA infection was seen in some limited regions several years ago, it has nowadays spread all over the country.

It is considered important as a countermeasure of the MRSA infection to make clean the environment within hospitals. Disinfectants such as ethanol, a quaternary ammonium salt (invert soap), sodium hypochlorite and an amphoteric surfactant are now generally used in a method for cleaning the environments such as floor of a hospital and disinfecting hands and fingers. However, the presence of other organic substance decreases the effect of the disinfectants because of non-selectivity of the disinfectants. Thus, it is the present situation that the use of these disinfectants is not satisfactory as the countermeasure of the MRSA infection. Therefore, it can be said that more effective materials are desired for preventing the MRSA infection.

### SUMMARY OF THE INVENTION

The present inventors have now found that an imidazole derivative is effective for preventing the MRSA infection, and have accomplished the present invention.

One object of the present invention is thus to provide a composition comprising an imidazole derivative having an anti-bacterial activity against MRSA.

Another object of the present invention is to provide fibrous products, paper products, sanitary goods which carry the composition.

The anti-bacterial composition according to the present invention comprises the compound represented by the general formula: 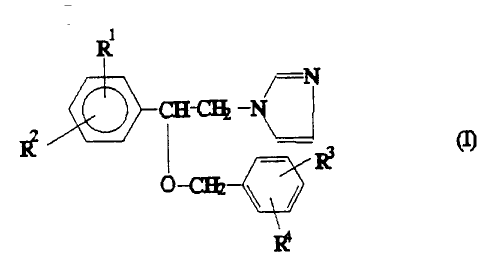
wherein R¹, R², R³ and R⁴ each represent independently a hydrogen atom or a halogen atom.

Specifically, according to the present invention, there provides fibrous products, paper products, sanitary goods, paints and dust absorbing oils having an anti-MRSA activity.

### DETAILED DESCRIPTION OF THE INVENTION

### Compound (I)

The compound represented by the general formula (I) is the compound described in Japanese Patent Publication No. 39664/1975.

In the general formula (I), R¹, R², R³ and R⁴ which are the substituents on the phenyl group or the benzyloxy group represent independently a hydrogen atom or a halogen atom. Preferred examples of the halogen atom include a fluorine atom, a chlorine atom, a bromine atom and an iodine atom, particularly a chlorine atom. If the substituent is a halogen atom, it is preferably positioned at the ortho- or para-position of the phenyl group or the benzyloxy group, although the position of the substituent is not limited to some of the ortho-, meta- or para-positions.

Preferred examples of the imidazole derivative having an anti-MRSA activity according to the present invention include
1-[2-(4-chlorobenzyloxy)-2-(4-chlorophenyl)-ethyl]imidazole,
1-[2-(2-chlorobenzyloxy)-2-(4-chlorophenyl)-ethyl]imidazole,
1-[2-benzyloxy-2-(2-chlorophenyl)-ethyl]imidazole,
1-[2-(4-chlorobenzyloxy)-2-(2-chlorophenyl)-ethyl]imidazole,
and
1-[2-(2-chlorobenzyloxy)-2-(2-chlorophenyl)-ethyl]imidazole.

These imidazole derivatives can be prepared according to the method described in Japanese Patent Publication No. 39664/1975.

### Composition/Fibrous Product etc.

The composition according to the present invention may be in the form of a prophylactic agent for the MRSA infection. Specifically, it may take any forms or applications adoptable as the prophylactic agent for infection with the proviso that an active ingredient is the compound of the general formula (I). For example, the compound of the formula (I) can be used as the raw chemical or as a variety of preparations such as a hydrated agent, an emulsion, powder or granules which are prepared by mixing the compound of the formula (I) with various liquid or solid carrier by the method generally used in the preparation of a prophylactic agent of infection and adding auxiliary agents such as a dispersant or an emulsifying agent to the mixture.

The liquid carrier for preparing these preparations preferably includes the one in which the compound of the formula (I) can be dissolved, or the one in which the compound of the formula (I) can be dispersed or dissolved with an auxiliary agent.

The liquid carrier includes for example water; a hydrocarbon solvent such as kerosine, toluene or a thinner; an alcohol such as methyl alcohol; an ether such as ethyl ether; a ketone such as acetone; an ester such as ethyl acetate; a polyhydric alcohol derivative such as polyethylene glycol; and an inorganic solvent such as silicon oil.

The solid carrier includes for example an inorganic compound such as sodium sulfate; a high melting compound such as a higher alcohol and a polyhydric alcohol; and paraffin wax.

While the concentration of the compound of the formula (I) as the effective ingredient in the preparations may be appropriately determined depending on an application way and site, it is generally in the range from about 1 to about 50% by weight, preferably from about 5 to about 20% by weight in a liquid form and in the range from about 1 to about 100% by weight, preferably from about 5 to about 20% by weight in a powder form. The preparation in a liquid form may be diluted with water to a concentration suitable for spraying just before use.

While the amount of the compound to be used varies depending on its applications, it is preferably in the range of 0.5 g/m² or more, more preferably 2 g/m² or more, of the reduced amount of the compound of the formula (I). In addition, the amount of the compound is desirably in the range which will not deleteriously affect the primary use of the goods or products to be treated while the activity of the compound is sustained with the passage of time.

The composition according to the present invention can be supported on the goods or products which are used or touched by a patient infected with MRSA and thus have potency of transmitting the MRSA infection. Such things include fibrous products such as a sheet, a covering, a carpet, a curtain, a surgical gown, a white robe, a mask and night clothes; paper products such as a wall paper, a paper towel and a paper cup; and sanitary goods such as a mop, a floor mat, a chemical dust cloth and a covering of a toilet seat.

The method for supporting the composition on the goods or products includes, but not limited to, a method of directly spraying the composition with an air-spray gun, a method of producing a anti-bacterially processing fibrous products with padding or exhausting, and a method of integral-molding in advance the composition into a resin pellet or the like. The amount of the compound of the formula (I) in the fibrous products, paper goods or sanitary products may be the same as that described above.

The composition according to the present invention may be in a form such as a dust adsorbing oil, a paint or an aerosol. The composition, for instance, may be prepared by blending such a substance with the compound of the formula (I) as a raw chemical or a formulation containing the compound of the formula (I) to give the anti-MRSA activity to the composition. In this case, the composition can be obtained by blending the compound of the formula (I) preferably in an amount from 0.005 to 5% by weight, more preferably from 0.02 to 2% by weight, with such a substance.

The composition and the fibrous products can be used in places where MRSA infection is foreseen to occur such as hospitals, nursery schools, kindergartens, primary schools or nursing homes.

### EXAMPLE

The present invention is further described in details with reference to the example and anti-bacterial activity tests set forth below, but it is not intended to be limited thereto.

### Example Preparation of 1-[2-(chlorobenzyloxy)-2-(2-chlorophenyl)-ethyl]imidazole

The preparation was carried out following to the scheme set forth below.

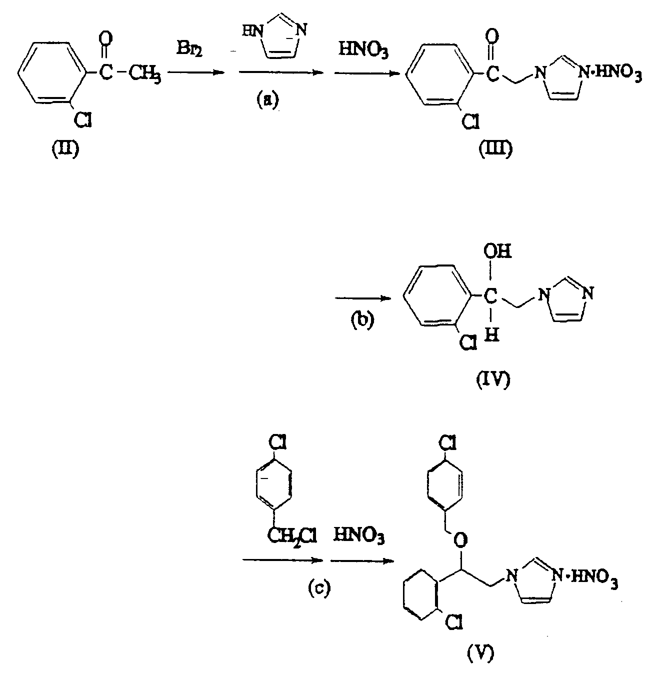

### (a) Synthesis of o-chlorophenacyl bromide and the formation of its imidazole derivative

To the solution of 30 ml of dioxane in 7.73 g of 2'-chloroacetophenone (Compound (II), 50 mmoles) was added dropwise 1.29 ml (0.5 equivalent) of Br₂ (M.W. 159.8, d = 3.102) under ice-cooling, and the mixture was stirred at 5°C for 2 hours. After the reaction was completed, 17.7 g (5.2 equivalents) of imidazole (M.W. 68.08) was dissolved in 30 ml of methanol and added dropwise to the reaction mixture under ice-cooling. After the addition was completed, the mixture was stirred overnight at room temperature. Chloroform and water were added to the reaction, and the mixture was separated. The organic phase was dried over magnesium sulfate and filtered, and the solvent was then removed by distillation. After 50 ml of acetone was added to the residue, 5 ml of fuming nitric acid was added dropwise and the mixture was stirred under ice-cooling. Crystallized solids were collected by filtration to give 7.79 g of crude crystals (yield 51%), which were further crystallized from 95% ethanol to give 4.4 g of Compound (III) (yield 31%).

### (b) Reduction of Compound (IV)

Compound (III) (4.25 g, 15 mmoles) prepared in the above step (a) was dissolved in 90% methanol, and 851 mg (1.5equivalents) of sodium borohydride (M.W. 37.83) was added portionwise to the solution. During the reaction, hydrogen gas was generated, and the reaction was exothermic. After the mixture was stirred at room temperature for 1 hour, acetone was added, and the resulting mixture was concentrated into a half of its volume. Water (10 ml) was added to the concentrate, which was adjusted to pH 2 with 5N HCl and then to pH 8 with 5N NaOH, and the mixture was stirred under ice-cooling. Crystallized solids were collected by filtration to give 3 g of Compound (IV) (yield 88%) as white powder.

### (c) p-chlorobenzylation

To the suspension of 0.48 g of 55% sodium hydroxide (2.2 equivalents) in 15 ml of dimethylformamide was added 1.1 g of Compound (IV) (5 mmoles) prepared in the above step (b) followed by 0.97 g of p-chlorobenzyl chloride (1.2 equivalents), and the mixture was stirred at room temperature for 5 hours. The reaction mixture was poured into ice-water and extracted with ethyl acetate. The organic phase was washed with saturated aqueous saline, dried over magnesium sulfate, and the solvent was removed by distillation. After the residue was dissolved in 10 ml of ethanol, 0.6 ml of fuming nitric acid was added, and the mixture was stirred under ice-cooling to give 1.3 g of the title compound (yield 63%) as deposited powder.

### Anti-bacterial activity test 1: Anti-bacterial activity

Minimum growth inhibiting concentration (MIC) of the compound represented by the general formula (I) was determined against some typical MRSA strains. The results are shown in the following table.

**Table 1**

| Anti-bacterial activity against MRSA MIC (µg/ml) | | | | | | | | | | |
|---|---|---|---|---|---|---|---|---|---|---|
| Compound of the formula (I) | | | | PRC-376 | S. aureus (MRSA) | | | | | |
| R¹ | R² | R³ | R⁴ | | -381 | -385 | -389 | -390 | -395 | -400 |
| p-Cl | H | p-Cl | H | 50 | 25 | 50 | 50 | 50 | 50 | 25 |
| p-Cl | H | o-Cl | H | 50 | 25 | 50 | 50 | 50 | 50 | 25 |
| o-Cl | H | H | H | 100 | 50 | 100 | 100 | 100 | 100 | 50 |
| o-Cl | H | p-Cl | H | 25 | 12.5 | 25 | 25 | 25 | 25 | 12.5 |
| o-Cl | H | o-Cl | H | 25 | 25 | 25 | 50 | 25 | 25 | 25 |

### Anti-bacterial activity test 2: Fibrous products

A cotton cloth was subjected to anti-bacterial processing with the compound of the general formula (I). The processing was carried out by applying a solution containing 0.05% of the compound of the general formula (I) and 1% of an organic silicone (fiber finish, Miki Riken) on the cotton cloth with the padding method. A cotton cloth (30 cm x 40 cm) was padded so that the pick-up was 70%, dried with hot air at 110°C for 3 minutes and further cured at 170°C for 1 minute. The anti-bacterial effect of the cotton cloth subjected to the anti-bacterial processing was evaluated with methicillin (DMPPC) resistant Staphylococcus aureus by the following method. The strains were selected on the basis of their resistances, and TY-5548 was selected as a higher resistant strain and TY-5552 as a lower resistant strain.

A 0.2 g sample of the cotton cloth was weighed, placed in a screw capped 30 ml vial and sterilized under pressure. The test strains had been pre-cultured and diluted with nutrient broth for inoculation to 5 - 30 x 10⁵ cells/ml. This culture medium was uniformly inoculated at an amount of 200 µl with a micropipette to the sample cloth, which was then incubated in an incubator at 37°C for 18 hours. After the incubation, 20 ml of sterilized physiological saline was added to the sample, which was stirred with a test tube mixer to disperse the microbial cells into the saline. The diluted dispersion prepared by the dilution method was spread and cultured over an agar plate. The colonies grown were counted to calculate the viable cells in the sample piece. The results with use of the Compound (V) are shown in Table 2.

**Table 2**

| | Y-5548 | | TY-5552 | |
|---|---|---|---|---|
| | Inoculated cells | After incubated | Inoculated cells | After incubated |
| Processed | 1.9 x 10⁵ | <2.0 x 10² | 1.4 x 10⁵ | <2.0 x 10² |
| Non-processed | 1.9 x 10⁵ | 6.2 x 10⁸ | 1.4 x 10⁵ | 1.5 x 10⁸ |

With respect to the sample subjected to the anti-bacterial processing, no colonies were detected. This meant that in the sample, viable cells were below the limit of sensibility (<2.0 x 10²) in the test. Such a processing successfully afforded the cell reducing rate of 99.9% or more of the inoculated cells. Thus, It is possible to prevent MRSA attached to a cotton cloth as clothing from further transmitting and thus to prevent the MRSA infection.

### Anti-bacterial test 3: Paper products

Anti-MRSA activity was afforded as described below to a wall paper on the assumption that the paper is used as a wall material of medical facilities such as a hospital room where there is a risk of infection from MRSA. An aqueous acrylic resin emulsion (NK Binder, Shin-Nakamura Kagaku) containing 0.1% of the compound of the general formula (I) was directly sprayed on the wall paper, which was then air-dried. The anti-bacterial activities of the wall paper thus treated were evaluated according to Anti-bacterial activity test 2, except that the culture inoculated contained 0.1% of a nonionic surfactant, Tween 80. The results with use of the Compound (IV) are shown in Table 3.

**Table 3**

| | Y-5548 | | TY-5552 | |
|---|---|---|---|---|
| | Inoculated cells | After incubated | Inoculated cells | After incubated |
| Processed | 5.0 x 10⁵ | <2.0 x 10² | 4.5 x 10⁵ | <2.0 x 10² |
| Non-processed | 5.0 x 10⁵ | 6.0 x 10⁸ | 4.5 x 10⁵ | 4.8 x 10⁸ |

With respect to the sample subjected to the anti-bacterial processing, no colonies were detected. This meant that in the sample, viable cells were below the limit of sensibility (<2.0 x 10²) in the test. Such a processing successfully afforded the cell reducing rate of 99.9% or more of the inoculated cell. Thus, It is possible to prevent MRSA attached to a wall pater from further transmitting and thus to prevent the MRSA infection.

### Anti-bacterial activity test 4: Dust adsorbing oil

Anti-MRSA activity was afforded to a dust adsorbing oil for a dust control mop as described below.

Only non-volatile ingredients were recovered from a dust adsorbing oil for a mop by spraying (manufactured by Yamazaki Sangyo), and incorporated with 0.1% of Compound (V).

A 200 µl of a suspension containing methicillin resistant Staphylococcus aureus (TY-5548, higher resistant bacterium) in a level of 1 x 10⁵ - 10⁴ cells/ml was smeared on a plate count agar, on which stainless steel cups having a diameter of 12 mm were placed to add 100 µl of the oil solution prepared above. The plate thus having inoculated the bacterium was left standing at 4°C overnight and then cultured at 37°C for 24 hours. The propagation of the bacterium under the cup was evaluated. As a result, propagation of the bacterium was observed with the control oil but no propagation was observed with the oil incorporated with the compound.

It is therefore possible to prevent the infection of MRSA adsorbed in the dust adsorbing oil by incorporating the oil with the compound according to the present invention.

### Anti-bacterial activity test 5 Paint

The compound of the general formula (I) was blended with a paint on the assumption that the paint is used around the medical facilities like in the case of Anti-bacterial activity test 3.

With an oil paint (manufactured by Shinto Toryo) 0.1% of the compound of the general formula (I) was mixed, and an appropriate dilution with thinner was spread over a stainless steel plate having a size of 3 cm x 3 cm and air-dried. The sample on which the film had been formed was sterilized with an alcohol and dried. The sample plate was placed in a glass dish, inoculated with 200 µl of the inoculation medium in Anti-bacterial activity test 3 (TY-5548, higher resistant bacterium) over the surface of the film and incubated at 37°C for 18 hours. After the incubation, 20 ml of a sterile physiological saline buffer was added to the medium to disperse the viable cells into the medium, and then the viable cells on the test piece were calculated in the same manner as in Anti-bacterial activity test 2. The result with the compound (V) are shown in Table 4.

**Table 4**

| | Y-5548 | |
|---|---|---|
| | Inoculated cells | After incubated |
| Processed | 1.4 x 10⁵ | <2.0 x 10² |
| Non-processed | 1.4 x 10⁵ | 1.4 x 10⁷ |

With respect to the sample subjected to the anti-bacterial processing, no colonies were detected. This meant that in the sample, viable cells were below the limit of sensibility (<2.0 x 10²) in the test. Such a processing successfully afforded the cell reducing rate of 99.9% or more of the inoculated cells. Thus, It is possible to prevent MRSA attached to such a painted surface from further transmitting and thus to prevent the MRSA infection.

## Claims

1. A composition for resisting methicillin resistant staphylococcus aureus (MRSA) comprising a compound represented by the general formula (I): 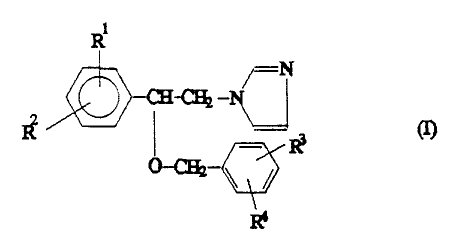 wherein R¹, R², R³ and R⁴ each represent independently a hydrogen atom or a halogen atom.

2. A composition according to claim 1, which is used as a prophylactic agent of the MRSA infection.

3. Fibrous products which carry the composition according to claims 1 or 2.

4. Fibrous products according to claim 3, wherein said fibrous products are selected from a sheet, a covering, a carpet, a curtain, a surgical gown, a white robe, a mask and night clothes.

5. Paper products which carry the composition according to claims 1 or 2.

6. Paper products according to claim 5, wherein said paper products are selected from a wall paper, a paper towel and a paper cup.

7. Sanitary goods which carry the composition according to claims 1 or 2.

8. Sanitary goods according to claim 7, wherein said sanitary goods are selected from a mop, a floor mat, a chemical dust cloth and a covering of a toilet seat.

9. A composition according to claim 1, wherein said composition is in a form selected from a paint, a dust adsorbing oil and an aerosol.
